# EUROPEAN PATENT SPECIFICATION

(11) **EP 2 277 400 B1**
(45) Date of publication and mention of the grant of the patent: **12.04.2017**
(21) Application number: 08757327.5
(22) Date of filing: 21.05.2008
(51) Int. Cl.: A43B 7/00, A61H 39/04, A61N 2/08, A43B 7/14, A43B 23/00, A61N 2/06, A43B 1/00

(54) **A MAGNETISM THERAPY SHOE**
MAGNETTHERAPIESCHUH
CHAUSSURE DE MAGNÉTOTHÉRAPIE

(30) Priority: 14.05.2008 CN 200810096996
(43) Date of publication of application: 26.01.2011
(73) Proprietor: Wu, Liji, Inner Mongolia 010011 (CN)
(72) Inventor: Wu, Liji, Inner Mongolia 010011 (CN)
(74) Representative: Algemeen Octrooi- en Merkenbureau B.V.
(86) International application number: PCT/CN2008/000981
(87) International publication number: WO 2009/137959

(56) References cited:
- EP-A1- 1 616 494
- CN-A- 1 087 248
- CN-A- 1 142 340
- CN-A- 1 391 857
- CN-A- 102 379 485
- CN-Y- 2 484 771
- CN-Y- 2 604 076
- US-A- 6 151 807

## Description

### Technical field

This invention relates to shoes having magnetotherapy functions, especially relates to leather shoes, cotton padded shoes, sandals, slippers and all other kinds of shoes.

### Background Art

Us 6,151,807 relates to a health care shoe, comprising: a sole having a plurality of magnet cavities specifically indented and distributed on an inner surface thereof and at least a medicine receiving cavity having at least a medicine unit received therein, wherein the medicine unit comprises a medicine layer therein and a bottom magnet element positioned thereunder so as to produce magnetic field around the medicine unit; a shoe body rigidly attached on top of the sole to define a foot chamber between the shoe body and the sole; a plurality of base magnet elements fittingly received in the magnet cavities respectively provided on the sole; and a plurality of upper magnet elements embedded inside the shoe body with respect to the positions of the base magnet elements, wherein the upper magnet elements are arranged to enable magnetic fields flowing between the base magnet elements and the upper magnet elements.

Modern medicinal theory considers that human diseases, whether they are diabetes, hypertensive, coronary heart disease, or various chronic diseases in the neck, the shoulders, the waist, the legs and etc., the pathogenies are the same although their diseased regions are different. They are all brought about by the blockade of the blood flow, which results in the hindrance of the microcirculation and hence in ischemia and anoxia. When problems of both ischemia and anoxia occur, the above-mentioned chronic diseases will befall. Therefore, all the chronic diseases are collectively called chronic diseases of the ischemic type.

The red cells, white cells and platelets in human blood all carry positive- or negative charges.

Therefore, when the blood of the whole human body circulates through the foot region in about every 30 seconds, the movement of the iron-containing red cells in the blood will cut through the magnetism lines of the huge magnetic field of the Earth, it will result in magneto-electric induction and bring about inductive currents. Having reinforced by the biological currents, the red cells transfer the biologic current carried with them to various kinds of the cells of the whole body and reinforcing the whole human body with sufficient supply of biologic current.

The design of the said shoes is based upon the above-mentioned physical principle about the effect of magnetic field of the Earth, to produce magnetic fields whose strengths are stronger than that of the Earth's in the shoes by appropriately installing magnets there.

As the blood flows through the human foot region, it passes through the magnetic field in the said shoes and cuts the magnetic line of force, then will engender inductive currents and joule heat effect, and cause the warming effect in the foot region. That is, the human body is sufficiently reinforced biologic currents.

In the course of walking with said kind of shoes put on, they will take magnetotherapy to the red cells, white cells and platelets in the blood flowing through the foot region, reinforce the human body with biological currents , enhance and improve circulation and microcirculation in the whole body and activate the cells. The shoes for magnetotherapy are suitable for diabetes and its complications, hypertension, coronary heart disease, bradycardia, cerebral infarction, cerebral thrombosis, sequel of cerebral haemorrhage, cervical spondylosis, lumbar spondylosis, scapulohumeral periarthritis, rheumatism, rheumatoid arthritis, gout, femoral head necrosis, weakness in the waist and legs, insomnia, astriction, hyperplasia of prostate gland, bronchitis, allergic asthma, allergic rhinitis, neurodermatitis, eczema, drug rash, anemia, gastritis, enteritis, various renal disease, gynaecopathia and all kinds of chronics of the ischemic type. The shoes can be used for the therapy of several diseases synchronously with high recovery rate, it is safe and practical, and it is easy to use.

The said shoes are safe and effective, it saves time without trouble by using them, because the sensitivity of the foot region to the magnetic field is higher than other parts of the human body, the effects of the said shoes are dependable, the implementation of the therapy is easy to carried out.

Use of the said shoes is easy: therapeutic effects are functioning anytime in the course of walking with the said shoes put on; the patients are wearing shoes and curing the disease at the same time, thereby decreasing the economic burden of the patients with one object having two functions.

Putting on the said shoes causes no feeling of discomfort and of pain from wound: the said shoes make the foot region feel warm and comfortable by joule heat effect, and feel strong in waist and legs. For those people who dread taking medicine, injection, acupuncture and operation, who fear pain and who are old or are feeble middle-aged as well as the underage, the said shoes are especially suitable.

### Summary of the invention

Reckoning with the shortcomings of the present technologies, the aim of this invention is to offer a kind of shoes for magnetotherapy, to promote unobstruction and vigorous blood circulation in the foot region, thereby to achieve the effects of treatment and prevention to various kinds of chronic diseases.

To realize the above-mentioned purposes, the present invention relates to a kind of shoes for magnetotherapy, according to the appended set of claims.

### Brief description of the figures

Fig. 1 is the stereogram of the shoes in the first preferable example of the shoes for magnetotherapy;
Fig. 2 is the planar graph of the soles in the first preferable example of the shoes for magnetotherapy;
Fig. 3 is the sectional view of the soles in the first preferable example of the shoes for magnetotherapy;
Fig. 4 is the stereogram of the soles in the first preferable example of the shoes for magnetotherapy;
Fig. 5 is the structural illustration of front upper in the first preferable example of the shoes for magnetotherapy;
Fig. 6 is the sectional view of front upper in the first preferable example of the shoes for magnetotherapy;
Fig. 7 is the structural illustration of rear- side upper in the first preferable example of the shoes for magnetotherapy;
Fig. 8 is the planar graph of the soles in the second preferable example of the shoes for magnetotherapy;
Fig. 9 is the sectional view of front upper in the second preferable example of the shoes for magnetotherapy;
Fig. 10 is the stereogram of the third example of the shoes for magnetotherapy;
Fig. 11 is the stereographic structural illustration of the soles in the third example of the shoes for magnetotherapy;
Fig. 12 is the sectional view of front upper in the third example of the shoes for magnetotherapy;
Fig. 13 and 14 are the stereographic structural illustration and sectional view of the soles in the fourth example of the shoes for magnetotherapy, respectively;
Fig. 15 is the sectional view of front upper and rear- side upper in the fourth example of the shoes for magnetotherapy.

### Detailed description of preferred embodiments

For better understanding of the shapes, structures and features in this invention, following is the detailed exemplification by means of preferable examples and referring to the figures. The practical proportions may be changed in the said figures for the sake of clarity of the structures in this invention and this should not be understood as restrictions to the claims of this invention.

### Example 1

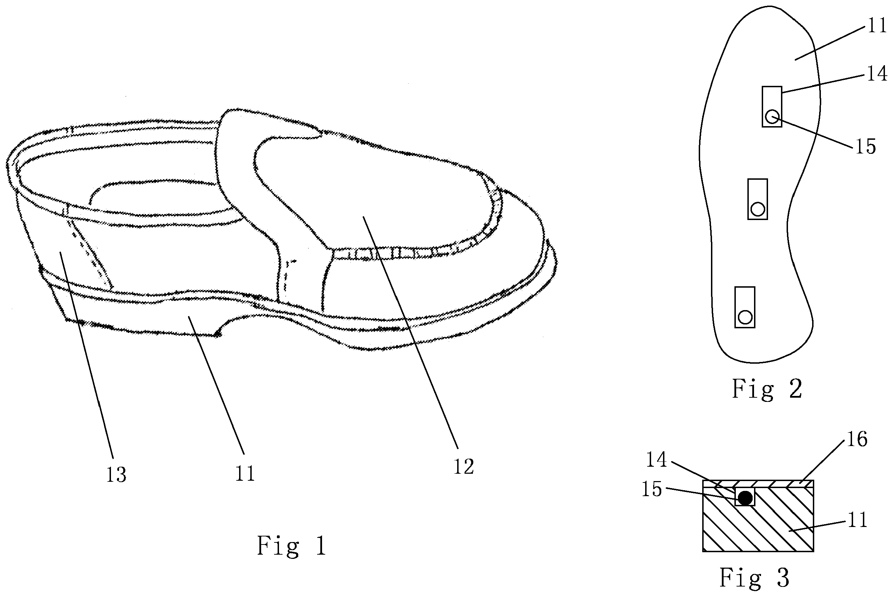

As shown in Fig. 1, that is the stereogram of the first preferable example of the shoes for magnetotherapy. Said shoes are of leather, which comprise the soles (11), the front uppers (12) and the rear- side upper (13) of the shoes. Magnets are installed in the soles (11), the front uppers (12) and the rear-side upper (13) of the shoes. The way they are installed will be described in detail in Fig. 2 to Fig. 7.

As shown in Fig. 2, 3 and 4, they are planar graph, sectional-view and stereographic illustrations of the soles (11) of the shoes for magnetotherapy, respectively. Said soles are of rubber or plastic materials, at least two grooves (14) are formed in the parts of the soles which are in contact with feet. Each one magnetic bead (15) is put into each of the grooves (14). In this example, the width of groove (14) is slightly wider than the diameter of the magnetic bead (15) in the direction of the shoe's width, and the length of groove (14) is larger than two times of the diameter of the magnetic bead (15) in the direction of the shoe's length, to facilitate the rolling of the magnetic beads in the grooves (14) along the direction of the shoe's length. As shown in Fig. 3, liners (16) are covered on the parts of the soles (11) which are in contact with the bottom of the feet to block the grooves (14) and to avoid the magnetic beads (15) escaping from the grooves (14).

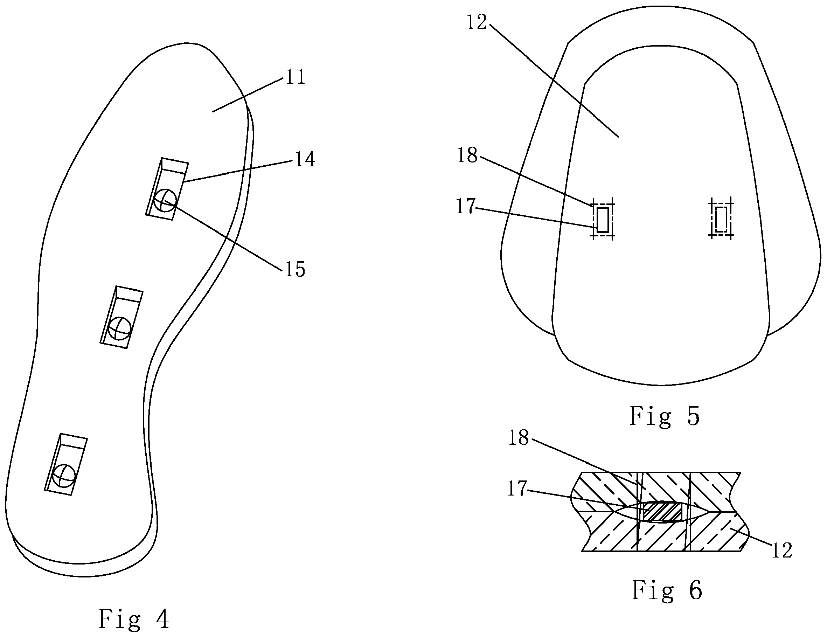

As shown in Fig. 5 and 6, they are the structural illustration and sectional view of the front uppers (12) of the shoes for magnetotherapy, respectively. Said front uppers of the shoes are comprised of vamp and inner layers. At least two soft or hard magnets (17) are stuck inside the front uppers. Said soft or hard magnets (17) are stuck into the place which is between the vamp and inner layers of the front uppers or can be further fixed by being sewn with thread (18) around them.

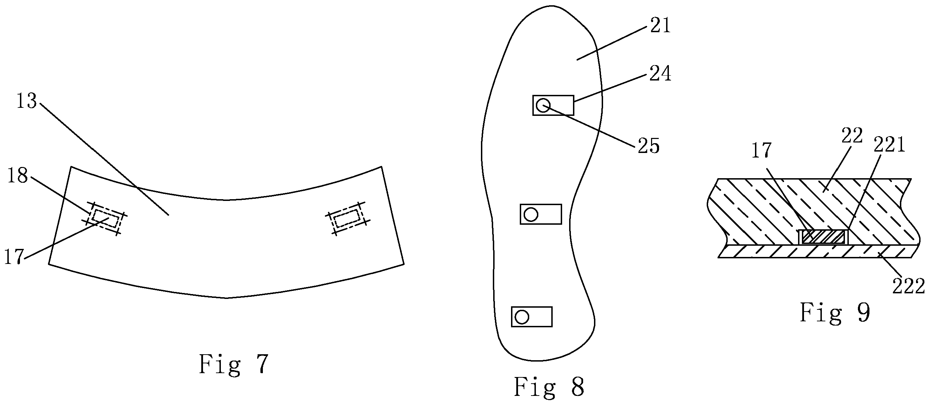

As shown in Fig. 7, that is the structural illustration of the rear-side upper (13). Said rear-side uppers (13) are comprised of vamp and inner layers. At least two soft or hard magnets (17) are stuck inside each rear-side upper (13). Said soft or hard magnets (17) are stuck into the place which is between the vamp and the inner layers of the rear-side uppers or can be further fixed by being sewn with thread (18) around them. As their structures are similar to those of the shoe front uppers (12), their sectional views are omitted.

As described above, magnets are all installed into the soles (11), the front uppers (12) and the rear-side upper (13) of the shoes for magnetotherapy. The magnetic beads (15) roll freely when the wearer walks. This will not only offer the external magnetic field to the feet of the wearer, but the position and strength of the magnetic field and the magnetic line of force will ceaselessly change with the free rolling of the magnetic beads, thereby the human body will be strengthened by strong or weak magnetic field in time.

### Example 2

As example 1, example 2 is about one kind of leather shoes, which comprise the soles (21), the front uppers (22) and the rear-side uppers (23) of the shoes. Magnets are installed in the soles (21), the front uppers (22) and the rear-side uppers (23) of the shoes.

As shown in Fig. 8, that is the planar illustrations of the soles (21) of the shoes for magnetotherapy. Said soles are of rubber or plastic materials. At least two grooves (24) are formed in the parts of the soles which are in contact with the feet. Each one magnetic bead (25) is put into each of the grooves (24). In this example, the width of said grooves (24) is slightly wider than the diameter of the magnetic bead (25) and the length of the grooves is larger than two times of the diameter of the magnetic bead (25). However, what differs from example 1 is said length of groove in this example can be either in the direction of the shoe's width or that extending in the direction of shoe's length, to facilitate the rolling of the magnetic beads. In consistent with the first example, a liner (16) is covered on the parts of the soles (21) which are in contact with the bottom of the feet, to block the grooves (24) and to avoid the magnetic beads (15) escaping from the grooves. The structure of the liner (16) is the same as that in example 1, it is not graphically illustrated here.

As shown in Fig. 9, that is the sectional structure illustration of the front uppers (22) of the shoes for magnetotherapy. Said front uppers (22) of the shoes are comprised of vamp and inner layers. At least two Shallow grooves (221) are formed in the parts of the front uppers (22) which are in contact with feet. At least two soft or hard magnets (17) are stuck inside each of the shallow grooves (221). To avoid direct contact of the soft or hard magnets with feet and rubbing them, protective layers (222) are installed outside the shallow grooves (221). Cotton cloth, plastic, leather, synthetic leather or any other soft materials can be used for making the said protective layers (222).

In the second example, the structure of the rear-side upper of the shoes is similar to the inner structure of the front uppers (22). At least two hallow grooves are placed in the parts of the rear-side upper of the shoes which are in contact with feet. At least two soft or hard magnets are stuck inside each of the shallow grooves. To avoid direct contact of the soft or hard magnets with feet and rubbing them, protective layers are covered outside the shallow grooves. Cotton cloth, plastic, leather, synthetic leather or any other soft materials can be used for making the said protective layers. As their structures are similar to those of the shoe front uppers in this example, the sectional view is omitted.

As described above, magnets are all installed into the soles (21) and front uppers (22) of the said shoes for magnetotherapy. The magnetic beads (25) inside the soles roll freely when the wearer walks.

### The derivative example of example 2

The grooves (24) in the parts of the soles (21) which are in contact with feet can also be set aslant. As this change of structure is simple and easy to understand, it is not graphically illustrated.

### Example 3

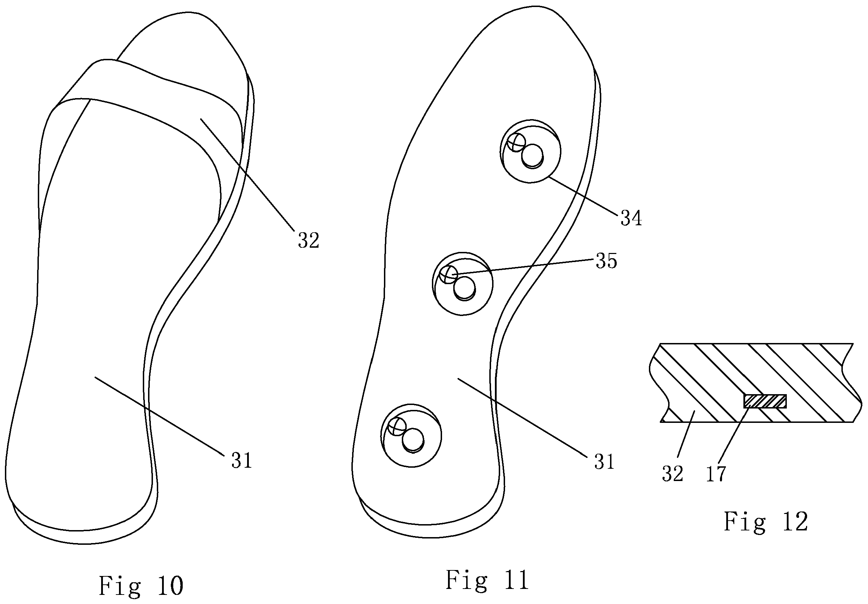

As shown in Fig. 10, that is the stereogram of the third preferable example of the shoes for magnetotherapy. Said shoes are slippers, which comprise the soles (31) and the front uppers (32). Magnets are installed in the soles (31) and the front uppers (32).

As shown in Fig. 11, that is the stereographic structural illustration of the soles (31). Said soles (31) are of rubber or plastic materials. At least two grooves (34) are formed in the parts of the sole which are in contact with the feet. Each one magnetic bead (35) is put into each of the grooves (34). In this example, said grooves (34) are of cirque shape. The magnetic beads (35) may roll freely inside the grooves (34). Liners are stuck on the parts of the soles (31) which are in contact with the bottom of the feet, to block the grooves (34) and to avoid the magnetic bead (35) escaping from grooves (34).

As shown in Fig. 12, that is the sectional view of the front uppers (32) of the shoes. Said front upper (32) can be made of leather, cotton cloth, rubber or plastic materials. At least two soft or hard magnets (17) can be stuck into the place which is between the vamp and inner layers of the front uppers. The soft or hard magnets (17) are stuck into the place which is between the vamp and inner layers of the front uppers or can be further fixed by being sewn with thread (18) around them.

As described above, magnets are all installed into the sole (31) and front upper (32) of the said shoes for magnetotherapy. The magnetic beads (35) inside the soles (31) roll circularly when the wearer walks.

### The derivative example of example 3

The shape of the grooves (34) in the parts of the soles (31) which are in contact with feet can also be elliptical, triangular, quadrangular, strip-shaped, cross- shaped. The effect of wearing them is essentially the same as example 3.

### Example 4

Example 4 is cotton padded shoes, which comprise the soles (41), the front uppers (42) and the rear-side upper of the shoes. Magnets are installed in the soles (41), the front uppers (42) and the rear-side upper of the shoes.

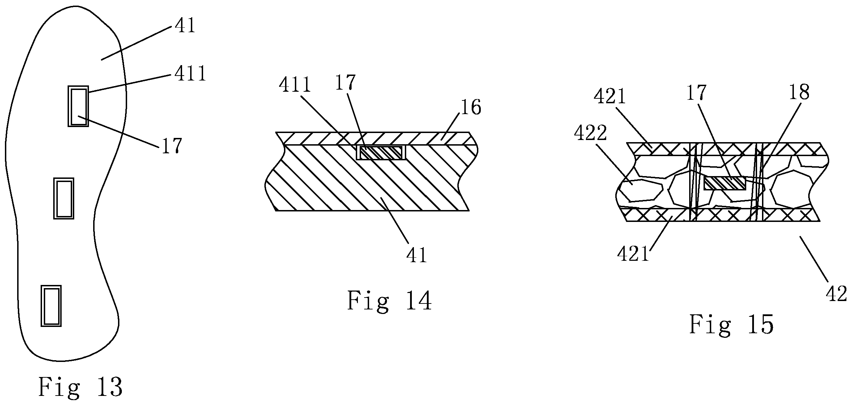

As shown in Fig. 13 and 14, they are the stereogram and sectional view of the soles (41), respectively. In the said soles (41), at least two shallow grooves (411) are formed in the parts of the soles which are in contact with the feet. At least two soft or hard magnets (17) (or a pair of soft or hard magnets (17) in parallel) are stuck into each of the shallow grooves (411). Protective layers (412) are covered on top of the shallow grooves (411) to avoid direct contact of the soft or hard magnets with the skin of the feet and rubbing them. Said protective layers (412) can be made of cotton cloth, plastic, leather, synthetic leather or any other soft materials.

The structures of front uppers (42) and rear-side upper are essentially the same in all examples and they are described collectively below. As shown in Fig. 15, the outer and inner surface of front uppers (42) and rear-side upper of the shoes are vamps and linings (421), respectively. Sponge or cotton wool materials (422) are padded between the vamps and linings (421). Soft or hard magnets (17) are placed inside the Sponge or cotton wool materials (422). Said soft or hard magnets (17) are stuck into the place which is between the vamps and linings or can be further fixed by being sewn with thread (18) around them.

The examples described above do not sum up all embodiments of this invention. On the basis of this invention, by carrying out various changes and combinations, for example, by doing technical redresses and recombinations of the designing and structures of this invention into various kinds of shoes, the technicians working in this field can produce different types of shoes for magnetotherapy without creative work. They can also do redresses and recombinations to the structures of the soles in some examples and those of the front uppers and rear-side upper of the shoes in other examples, and exchange the hard magnet for the soft, vice versa. These are all easy to think up and implement, nevertheless, these are all within the claim range of this invention.

The above description is illustrative and not limit to the scope of this invention. The technicians in this field understand that many modification and changes and equivalents can be done without deviating from the essence and scope of the claims of this invention, nevertheless, these are all within the protective range of this invention.

Finally, the beneficial effects of this invention are described in accordance with modern medicinal theory.
1. When biological current is abundant, bone density will increase and the hematopoietic function of the marrow stem cells will be strengthened.
   The skeleton is the framework of human body, marrow stem cells are the hematopoietic factory for producing red cells, white cells and platelets.
   Putting on the said shoes, the red cells will be reinforced plentifully with biological current, the velocity at which they carry oxygen, curl deformation and move helically forward will increase.
   The red cells will dredge swiftly the capillary vessel bed inside bones and endosteum, resulting in unobstructed, vigorous and smooth blood circulation in bones and endosteum.
   Thereby essentially eliminating the problems of microcirculation obstruction inside the bones and endosteum, as well as eliminating the problems of ischemia and anoxia.
   When the blood circulation inside the bone is unobstructed, vigorous and smooth, the bone will obtain ample supply of ossein fiberbunching and vitamin A, D and C, to promote the formation of bone matrix.
   In this way, precipitation of the osteoid and calcium salts onto the bone matrix can be facilitated, bone density increased, and the waists and the legs of the wearers strengthened. When the wearers go upstairs or walk a long way, they will not feel tired and lack of strength without sore waist and aching legs.
   The problem of clonus frequently occurred in the middle aged and the old is thereby being eliminated.
   As the red cells dredge capillary vessel bed inside bones and endosteum, they transfer the biologic current they carry with them to the marrow stem cells.
   The latter will be supplied with ample amount of biologic current, as well as blood and oxygen. The hematopoietic function of the marrow stem cells will be strengthened, producing mature red cells, white cells as well as platelets in time, and keeping the numbers of these blood cells at normal amounts.
   Putting on said shoes, it especially keeps the biological functioning of the bones and marrow stem cells of the middle aged and the old at an active and thriving status.
2. When the biologic current is abundant, the wearers' physical energy will be strengthened, their waists and legs will be forceful.

The majority of the middle-aged and the old have the problem of feebleness in their waists and legs, as well as the difficulty in walking and going upstairs.

After putting on the said shoes for 10 days, however, the blood circulation in their kidneys and parts of their body such as waists and legs will be unobstructed, vigorous and smooth, their physical energy strengthened, their waists and legs forceful.

After putting on the said shoes for 30 days, the problem of the feebleness of the waist and legs of each middle-aged and the old is essentially eliminated.

Even though for those as old as 70 to 80 years, the blood circulation in parts of their body such as waists and legs will be unobstructed, vigorous and smooth after putting on the said shoes for magnetotherapy. With their supply of blood and oxygen ensured, they are forceful and agile in their waists and legs.

Every middle-aged and the old who put on the said shoes are capable of walking incessantly for more than 5 hours but not feel tired and never need stop to rest.

If, the young who are about 20-year old were made to have the 5-hour walking, most of them could be too tired to continue walking when they just finish the half distance. Besides, they also need to have a rest during the walking.

That is because that the young men's biological currents are not as such sufficient as that of the old who put on the said shoes.

We can see from the above all, as long as to reinforce the biological currents to the human, it will make a huge enhancing to the physical energy of the middle-age and the old. Furthermore, the physical energy of the middle-age and the old will be more than that of the young men.

When the middle-aged and the old put on the said shoes, the red cells and white cells will be supplied with abundant reinforcement of biologic current, their physical energy and immunizing power will be thereby greatly increased.

The above description of the practical effects that the physical energy is strengthened and waists and legs are forceful after putting on the said shoes is experienced by everybody who tested the said shoes.

The above description is illustrative and not limit to the scope of this invention, which is defined by the appended claims.

## Claims

1. A kind of shoes for magnetotherapy, wherein said shoes comprising soles (11, 21, 31, 41), front uppers (12, 22, 32, 42) and/or rear-side uppers (13, 23), are selected from non-padded shoes, padded shoes, sandals, slippers and all kinds of shoes, wherein there are at least two grooves (14, 24, 34) on each of said soles (11, 21, 31, 41) and one magnetic bead (15, 25, 35)is placed in each of said grooves (14, 24, 34); **characterized in that**,
at least two shallow grooves (221, 411) are formed in the place of contact of soles (11, 21, 31, 41) and foot, wherein either one soft or hard magnet (17)or a pair of soft or hard magnets (17) arranged in parallel are stuck into each of the shallow grooves (221, 441); a layer of liner (16) covers on top of the shallow grooves (221, 411); at least two soft or hard magnets (17) or a pair of soft or hard magnets (17) arranged in parallel are placed between the vamp and the inner of both front uppers (12, 22, 32, 42) and rear-side uppers (13, 23) of said shoes; said soft or hard magnets (17) are stuck with glue or are fixed with thread (18) being sewn around them, wherein said magnetic bead (15, 25, 35) rolls freely in each of said grooves (14, 24, 34) when the wearer walks,

2. The shoes for magnetotherapy according to claim 1, wherein said grooves (14, 24, 34) are designed into optional depth and various shapes selected from annular, elliptical, triangular, quadrangular, strip, cross and other shapes, said grooves (14, 24, 34) are installed lengthily, laterally or slantingly or in any other orientation, the length of the groove (14, 24, 34) is two times larger than the diameter of the magnetic bead (15, 25, 35)and the width of the groove (14, 24, 34) is larger than the diameter of the magnetic bead (15, 25, 35)to facilitate the rolling of the magnetic bead (15, 25, 35)in said groove (14, 24, 34).

3. The shoes for magnetotherapy according to any one of the preceding claims, wherein optional amount of magnetic beads, magnetic slices, magnetic columns or other shape magnets are installed into the grooves (14, 24, 34, 221, 411) in any places of front uppers (12, 22, 32, 42), rear-side uppers (13, 23) and soles (11, 21, 31, 41) of the shoes.

4. The shoes for magnetotherapy according to any one of the preceding claims, wherein any magnets (14, 24, 34, 17) installed into said shoes are replaced with other kinds of magnetic materials with various specifications, different shapes and volumes selected from slices, strips and columns.

5. The shoes for magnetotherapy according to any one of the preceding claims, wherein said shoes contain at least two single chip magnets, or double chips magnet arranged in parallel.

6. The shoes for magnetotherapy according to any one of the preceding claims, wherein the magnetic beads (15, 25, 35) or hard magnets (17) placed in said shoes are changed into soft magnets (17) and vice versa, thereby changing into other types of shoes for magnetotherapy.

## Patentansprüche

1. Art von Schuhen zur Magnettherapie, wobei die Schuhe, die jeweils eine Sohle (11, 21, 31, 41), vorderes Obermaterial (12, 22, 32, 42) und/oder rückseitiges Obermaterial (13, 23) umfassen, aus nicht gepolsterten Schuhen, gepolsterten Schuhen, Sandalen, Pantoffeln und allen Arten von Schuhen ausgewählt sind, wobei sich an jeder der Sohlen (11, 21, 31, 41) wenigstens zwei Vertiefungen (14, 24, 34) befinden und in jeder der Vertiefungen (14, 24, 34) ein Magnetkügelchen (15, 25, 35) platziert ist; **dadurch gekennzeichnet, dass**
wenigstens zwei flache Vertiefungen (221, 411) an der Stelle des Kontakts von Sohle (11, 21, 31, 41) und Fuß gebildet sind, wobei entweder ein weicher oder harter Magnet (17) oder ein parallel angeordnetes Paar weicher oder harter Magnete (17) in jede der flachen Vertiefungen (221, 411) hineingesteckt ist; eine Lage Futter (16) die flachen Vertiefungen (221, 411) obenauf bedeckt;
wenigstens zwei weiche oder harte Magnete (17) oder ein parallel angeordnetes Paar weicher oder harter Magnete (17) zwischen dem Blatt und der Innenseite sowohl des vorderen Obermaterials (12, 22, 32, 42) als auch des rückseitigen Obermaterials (13, 23) der Schuhe platziert sind; die weichen oder harten Magnete (17) mit Kleber befestigt oder mittels um sie herum vernähtem Faden (18) fixiert sind, wobei das Magnetkügelchen (15, 25, 35) in jeder der Vertiefungen (14, 24, 34) frei rollt, wenn der Träger läuft.

2. Schuhe zur Magnettherapie nach Anspruch 1, wobei die Vertiefungen (14, 24, 34) in beliebiger Tiefe und mit verschiedenen Formen gestaltet sind, die aus ringförmig, elliptisch, dreieckig, viereckig, Streifen-, Kreuz- und anderen Formen ausgewählt sind, die Vertiefungen (14, 24, 34) längs, seitwärts oder schräg oder in irgendeiner anderen Ausrichtung angebracht sind, die Länge der Vertiefung (14, 24, 34) zwei Mal größer ist als der Durchmesser des Magnetkügelchens (15, 25, 35) und die Breite der Vertiefung (14, 24, 34) größer ist als der Durchmesser des Magnetkügelchens (15, 25, 35), um das Rollen des Magnetkügelchens (15, 25, 35) in der Vertiefung (14, 24, 34) zu erleichtern.

3. Schuhe zur Magnettherapie nach einem der vorhergehenden Ansprüche, wobei eine beliebige Menge an Magnetkügelchen, Magnetscheiben, Magnetstäben oder Magneten anderer Form in den Vertiefungen (14, 24, 34, 221, 411) an beliebigen Stellen des vorderen Obermaterials (12, 22, 32, 42), des rückseitigen Obermaterials (13, 23) und der Sohle (11, 21, 31, 41) der Schuhe angebracht sind.

4. Schuhe zur Magnettherapie nach einem der vorhergehenden Ansprüche, wobei jede Magnete (14, 24, 34, 17), die in den Schuhen angebracht sind, durch andere Arten magnetischer Materialien mit verschiedenen Spezifikationen, unterschiedlichen Formen und Volumina, ausgewählt aus Scheiben, Streifen und Stäben, ersetzt sind.

5. Schuhe zur Magnettherapie nach einem der vorhergehenden Ansprüche, wobei die Schuhe wenigstens zwei Magnete mit einem Stück oder einen Magnet mit zwei parallel angeordneten Stücken umfassen.

6. Schuhe zur Magnettherapie nach einem der vorhergehenden Ansprüche, wobei die in den Schuhen platzierten Magnetkügelchen (15, 25, 35) oder harten Magnete (17) gegen weiche Magnete (17) getauscht sind und umgekehrt, wodurch die Schuhe in andere Arten von Schuhen zur Magnettherapie geändert sind.

## Revendications

1. Genre de chaussures pour magnétothérapie, où lesdites chaussures comprenant des semelles (11, 21, 31, 41), des tiges avant (12, 22, 32, 42) et/ou des tiges de côté arrière (13, 23), sont sélectionnées à partir de chaussures non rembourrées, de chaussures rembourrées, de sandales, de pantoufles et de tout genre de chaussures, où il existe au moins deux rainures (14, 24, 34) sur chacune desdites semelles (11, 21, 31, 41) et une bille magnétique (15, 25, 35) est placée dans chacune desdites rainures (14, 24, 34) ; **caractérisées en ce qu'**au moins deux rainures peu profondes (221, 411) sont formées à la place du contact entre les semelles (11, 21, 31, 41) et le pied, où soit un aimant souple ou dur (17) soit une paire d'aimants souples ou durs (17) agencé(s) en parallèle est/sont collé(s) dans chacune des rainures peu profondes (221, 441) ; une couche de doublure (16) recouvre la partie de dessus des rainures peu profondes (221, 411) ;
au moins deux aimants souples ou durs (17) ou une paire d'aimants souples ou durs (17) agencés en parallèle sont placés entre la claque et l'intérieur à la fois des tiges avant (12, 22, 32, 42) et des tiges de côté arrière (13, 23) desdites chaussures ; lesdits aimants souples ou durs (17) sont collés avec de la colle ou sont fixés avec un fil (18) qui est cousu autour d'eux, où ladite bille magnétique (15, 25, 35) roule librement dans chacune desdites rainures (14, 24, 34) lorsque le porteur de chaussure marche.

2. Chaussures pour magnétothérapie selon la revendication 1, dans lesquelles lesdites rainures (14, 24, 34) sont conçues selon une profondeur facultative et diverses formes choisies parmi des formes annulaires, elliptiques, triangulaires, quadrangulaires, en bandes, en croix et autres, lesdites rainures (14, 24, 34) sont installées de façon longitudinale, latérale ou oblique ou dans toute autre orientation, la longueur de la rainure (14, 24, 34) est deux fois plus grande que le diamètre de la bille magnétique (15, 25, 35) et la largeur de la rainure (14, 24, 34) est supérieure au diamètre de la bille magnétique (15, 25, 35) pour faciliter le roulement de la bille magnétique (15, 25, 35) dans ladite rainure (14, 24, 34).

3. Chaussures pour magnétothérapie selon l'une quelconque des revendications précédentes, dans lesquelles une quantité facultative de billes magnétiques, de tranches magnétiques, de colonnes magnétiques ou d'autres aimants conformés est installée dans les rainures (14, 24, 34, 221, 411) dans n'importe quel emplacement des tiges avant (12, 22, 32, 42), des tiges de côté arrière (13, 23) et des semelles (11, 21, 31, 41) des chaussures.

4. Chaussures pour magnétothérapie selon l'une quelconque des revendications précédentes, dans lesquelles tous les aimants (14, 24, 34, 17) installés dans lesdites chaussures sont remplacés par d'autres genres de matériaux magnétiques ayant diverses spécifications, des formes et volumes différents choisis parmi des tranches, des bandes et des colonnes.

5. Chaussures pour magnétothérapie selon l'une quelconque des revendications précédentes, dans lesquelles lesdites chaussures contiennent au moins deux aimants à puce unique, ou des aimants à deux puces agencés en parallèle.

6. Chaussures pour magnétothérapie selon l'une quelconque des revendications précédentes, dans lesquelles les billes magnétiques (15, 25, 35) ou les aimants durs (17) placé(e)s dans lesdites chaussures sont transformé(e)s en aimants souples (17) et vice versa, ce qui permet de se transformer en d'autres genres de chaussures pour magnétothérapie.
